# EUROPEAN PATENT APPLICATION

(11) **EP 1 136 510 A1**
(43) Date of publication of application: **26.09.2001**
(21) Application number: 01302709.9
(22) Date of filing: 23.03.2001
(51) Int. Cl.: C08G 63/08, A61L 27/14, A61L 27/18, B29C 51/00

(54) **Thermoformed bioabsorbable polymers for use in medical devices**

(30) Priority: 24.03.2000 US 534892
(71) Applicant: ETHICON, INC., Somerville, NJ 08876 (US)
(72) Inventor: Datta, Arindam, Hillsborough, NJ 08876 (US); Siegmann, Arnon, Haifa 34791 (IL)
(74) Representative: Mercer, Christopher Paul

(57) **Abstract**

We have developed thermoformed bioabsorbable polymer preforms that are oriented in at least one axis (preferably two axes that are orthogonal) that are suitable for making into medical devices. This preforms are formed from bioabsorbable polymeric sheets that are heated, shaped against a three dimensional mold to impart orientation to the polymeric sheets and cooled to retain at least part of the orientation.

## Description

### Field of the Invention

The field of art to which this invention relates is biocompatible polymers, more specifically, medical devices made from biocompatible and/or absorbable polymer. Specifically, absorbable polyester preforms are suitable for being formed into orthopedic devices for use in the fixation of bone and cartilage, especially hard tissue of the cranium, face and other plastic and reconstructive procedures.

### Background of the Invention

Fracture fixation is a common surgical operation obtained by attaching an orthopedic reinforcing rod, plate or cage to a fractured bone so that the broken ends may be stabilized in order to promote fusion and consequent healing. Metallic orthopedic implants are currently used for fracture fixation due to their high stiffness and strength that stabilize the fracture site during tissue healing. Metallic implants are non-isotropic, therefore can generally stabilize deformations and stresses in more than one direction. However, the robust nature of metallic orthopedic implants creates several post-operative issues. Since metallic orthopedic implants are much stiffer than bones, metal implants become the primary load-bearing member thereby protecting the bone from stress. Unfortunately, bone tissue that is stress shielded will weaken over time. If the metal implant is not removed, the extended stress shielding will eventually, result in bone atrophy through decalcification or osteoporosis. Therefore, it is often necessary to perform a second surgical procedure to remove metal implants after the bone tissues have healed.

The use of bioabsorbable materials for fracture fixation has the potential to eliminate the necessity of a second operation and help alleviate the negative effect of stress shielding. Specifically designed bioabsorbable materials can have mechanical properties that begin to approach those of certain types of bone in some applications and are strong enough to stabilize the fracture. With time, the bioabsorbable material implant gradually decreases its stiffness and strength due to biodegradation. At the same time, the bone fracture starts to heal. During the course of these two overlapping events, the mechanical stresses are gradually transferred from the degradable bioabsorbable implant to the healing bone tissue.

Bioabsorbable polymeric materials have also been used in the form of pins, rods, anchors and staples for a variety of medical applications including orthopedic devices. Bioabsorbable orthopedic devices are usually made by injection molding or extrusion process. However, the relatively low stiffness and strength of bioabsorbable devices prepared by these processes, as compared with metallic orthopedic devices, have limited the use of bioabsorbable orthopedic devices to low-load bearing applications or non-load bearing applications.

A number of US patents such as US patent numbers 4,905,680; 5,057,111; 5,275,601; 5,290,281; 5,607,427; and 5,569,250 describe absorbable plating systems. These plates are compression molded or injection molded and in a number of instances subsequently machined. Bioabsorbable plates made by injection or compression molding suffer from the shortcoming that either they are not oriented as in compression molding or they are oriented in one direction only as is the case for injection molding. Because the plates are compression molded or injection molded, they have relatively low stiffness and strength values. Owing to complex flow inside the mold and thermal relaxation, the orientation developed both during compression and injection process, makes the absorbable devices suitable primarily for use in low-load bearing applications or non-load bearing applications. Since the forming or deformation temperatures are high, a substantial part of orientation is lost due to relaxation resulting in lower mechanical properties. The other principle drawback arises from the fact that even in injection molded plates; the somewhat higher properties are mainly in the principal material flow direction. Thus, these absorbable plates may not be suitable for healing those fractures in which the resistance to deformation may occur in more than one direction specifically for higher loads. It is also difficult to design or engineer high orientations in different directions in device made by injection molding.

A number of patents (such as US patent numbers 4,898,186; 5,227,412 and 4,968,317) describe methods of making pins, screws and plates for orthopedic applications with material properties higher than those obtained by injection molding or compression molding. In all of these patents, the bioabsorbable polymer is first melt processed by injection molding, compression molding or extrusion, into a preform. The preform is then heated to an elevated temperature (in most cases above the glass transition temperature of the polymer) and is drawn under uniaxial deformation mode. This method produces devices with bending modulus, flex modulus or flex strength higher than those of the preform, but the property enhancements are limited to only the draw direction. Products made using these processes have not demonstrated property enhancements in directions other than the principal draw direction.

Considerable effort has been applied towards increasing the stiffness and strength of bioabsorbable materials by using various composite technologies. One method of improving the stiffness and strength of bioabsorbable materials has been to make reinforced composite materials. Composite materials generally incorporate a strong and stiff non-absorbable inorganic structural fibers or particles, made from carbon or glass, as reinforcing agents in a bioabsorbable polymeric matrix. The disadvantage of these approach is that the non-absorbable fibers stay behind in the body tissue after the bioabsorbable polymer has been absorbed by the body and in the long run, may cause tissue reaction or other undesirable effects, such as unwanted migration.

Composites may also be prepared by incorporating inorganic bioabsorbable glass or ceramic reinforcement such as fibers or particles in bioabsorbable polymer matrix. However, lack of fiber-matrix interfacial bonding leads to poor load transfer mechanism between the fiber and the incompatible matrix. Poor interface problems are accentuated when implants are placed in the human body and may cause the implant to fail prematurely.

Reinforced bioabsorbable composites have also been made by adding a bioabsorbable reinforcing fiber to a bioabsorbable polymer matrix. The reinforcing polymer fibers are usually stiffer and stronger. Similarly, highly drawn fibers of polylactide (PLA) or polyglycolide (PGA) can be fused to develop bioabsorbable polymeric device with increased stiffness and strength. Unfortunately, the consolidation or the melting temperature of the matrix usually causes the biocompatible organic fibers to partially relax their molecular orientation, thereby losing their strength and stiffness, which consequently affect the properties of the composite. Thus the efforts to provide bioabsorbable for orthopedic load bearing applications has not been entirely successful.

In an unrelated field of polymer processing technology, researchers have described a number of bioabsorbable thin films have been made from polylactic acid and its copolymers. Generally these thin films were biaxial oriented to improve their performance for packaging applications. Some of these films are described in patents numbers US 5,443,780; JP 09025345; JP10138433; JP09174674; JP09157408 and JP07205278. Biaxially orienting these thin films was generally necessary to reduce heat shrinkage and improve dimensional stability of these films for use in containers and blister packages designed for hot food and beverages.

However, no one has described bioabsorbable plates for orthopedic load bearing applications. Additionally, processing methods for making thick bioabsorbable plates (i.e. greater than 0.3 mm) for machining into orthopedic load bearing applications have also not been described.

Some documents in the literature do generally describe using thermoforming processes to impart biaxial properties to articles made from non-absorbable polymers (i.e. US 4927690 and BE 894214) for non-medical uses. However, there have been no description or suggestion that thermoforming would be an appropriate method for manufacturing bioabsorbable devices.

It is the objective of the present invention to provide bioabsorbable materials in the form of thick plates with enhanced properties in more than one direction, that can be used for fracture fixation, and a process or method to produce these materials from which the plates will be machined.

### Summary of Invention

We have discovered a thermoformed bioabsorbable polymer preform having at least one axis (preferably two axes) of orientation for medical use (preferably with two axes being orthogonal).

One embodiment of the present invention relates to a thermoformed bioabsorbable medical device (preferably plates), having a minimum thickness greater than 0.3 mm (0.012 inches) and have multi-axial orientation.

In a further embodiment of the present invention, we have discovered a bioabsorbable device in the form of plates, with mechanical properties higher than those obtained by injection molding in at least two directions and these two directions are preferably orthogonal to each other. The devices can be curved or straight.

Another embodiment of the present invention provides a method to fabricate these devices with higher properties in more than one direction by using a unique forming process i.e. thermoforming. The devices are formed from sheets, that are thicker than the final device and are formed by controlled preheating of the sheet and shaping the heated sheet against a three dimensional mold. The stretching that is imparted to the original sheet is controlled and provides molecular orientation in multiple directions. In the case of semi-crystalline polymers the stretching also provides crystalline orientation.

The foregoing and other features and advantages of the invention will become more apparent from the following description and accompanying examples.

### Description of the Drawings

Figure 1 illustrates one embodiment of the present invention, plug assisted vacuum forming. This figure illustrates the initial set up for beginning plug assisted vacuum forming. In the initial set up, sheet 2 is held in clamps or frame 4 and positioned between plug 12 and mold 10.
Figure 2 illustrates the second step in plug assisted vacuum forming. In this step of the process the clamp or frame 4 and mold 10 are moved so that a seal is formed between the top of mold 8 and sheet 2. The sheet 2 is heated and then it is pressed towards mold 10 by plug 12. Air is being removed from the mold through passages 16 after the seal has been formed. The removal of the air causes sheet 2 to be pulled into the interior 14 of mold 10.
Figure 3 illustrates the final position of sheet 2 in plug assisted vacuum molding process. Sheet 2 has been completely stretched (drawn) against mold 10.
Figure 4 illustrates another embodiment of the present invention, matched mold vacuum forming. The starting set up for this process is illustrated in this figure. In the initial set up sheet 2 is positioned between a male mold 11 (which can also act as a plug) and female mold 10.
Figure 5 illustrates the final position of sheet 2 in the matched mold vacuum forming. The sheet 2 was heated, then it was pressed toward the female mold 10 by male mold 11 and air was removed from the mold through passages 16.

The removal of air causes sheet 2 to be pulled into the interior 14 of mold 10. Sheet 2 has been formed between male mold 11 and female mold 10.

### Detailed Description of the Invention

What follows is a description of the devices, preferably orthopedic devices, and methods to manufacture them by a process that will provide organic bioabsorbable devices with superior mechanical properties such as, tensile modulus and tensile strength, in more than one direction that are preferably orthogonal to each other. The devices of the present invention will have a thickness above 0.3 mm and be produced by thermoforming. The devices will be produced by controlling the stretching or drawing (orientation of the molecular structure) and by controlling the thermal histories and thereby controlling the structure and the properties of the devices. The devices are formed at elevated temperatures at which the material from which they are made is in a rubbery state, therefore, are readily deformable and can be more highly oriented. The high orientation is retained by rapidly cooling the heated device by first forming against a cooler mold and can be subsequently further cooled by forced convection. Owing to the unique multi-axial deformation modes of thermoforming, the devices can be stretched to provide molecular orientation multiple directions. The stretching can depend on the mold design. Thermal history can be controlled to provide the desired initial crystallinity, and stretching and thermal history can be controlled to provide orientation to the devices. Devices with higher modulus, higher strength and improved fatigue resistance are thus created.

The starting material of the present invention is generally formed by use of compression molding a bioabsorbable polymer, in form of pellets, in a press preferably under a dry inert environment (under nitrogen, argon, etc.) or under vacuum. The thermoplastic pellets may be transformed and consolidated into a sheet of pre-determined thickness under appropriate temperatures, pressures and residence times using conventional processing procedures which are well known to those skilled in the art. The materials that form the sheet are generally either amorphous or semi-crystalline bioabsorbable polymers and these preformed sheets are subsequently thermoformed.

Alternately, it is possible to form the preform sheets by extruding a thick sheet using an extruder, an appropriate sheet die and belt conveying system to draw, control the draw and support the continuous sheets thus formed. Yet another method to form the pre-form sheet is by injection molding.

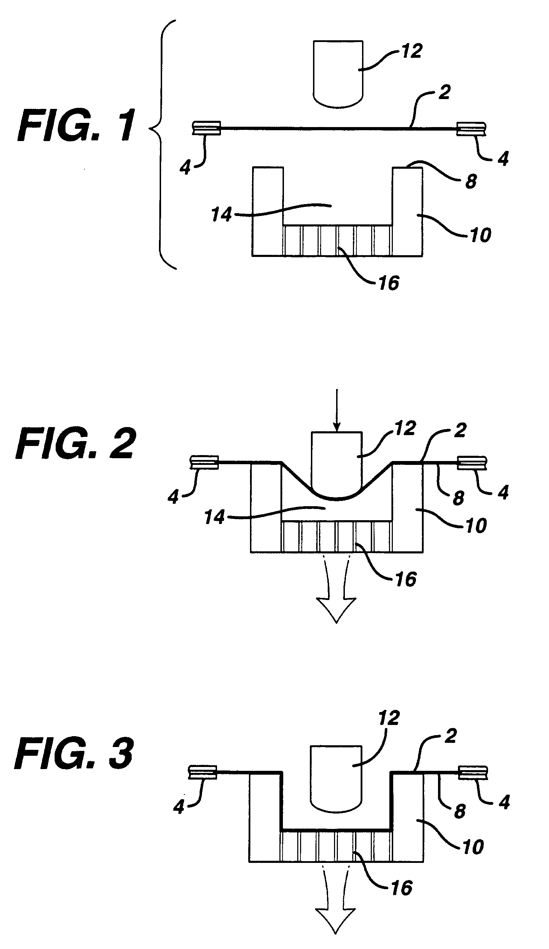

In the first step of thermoforming, the polymeric sheets are constrained between two clamps or frames. The entire assembly, consisting of clamp and preform sheet, is moved in an oven that is heated by a top and/or a bottom bank of heaters. The temperature of the heaters and the residence time controls the temperature of the sheet. The heated sheet is removed from the oven and is in a rubbery state. The rubbery sheet is then sealed and restrained against a rim of a female mold and the rubbery sheet can be pre-stretched slightly with a heated or unheated plug before the final forming of the sheet in the female mold cavity. Vacuum is then applied to pull or draw the slightly pre-stretched sheet against the female mold surface until it stops drawing. The heated rubbery sheet is oriented as it stretched and cools during stretching. When the sheet has finally been stretched against the mold, whose temperature can be controlled, the plug has no contact with the mold or the sheet. The vacuum is applied to the mold for until the stretching is complete. Generally the vacuum is applied until the stretched sheet cannot be deform any more. The cooling process can be continued by forced convection methods such as blowing air at ambient temperature to retain the orientation that has been developed in the device. A simplified embodiment of this process is illustrated in Figures 1-3. The molds that are used in the process of course can be of numerous shapes to facilitate providing the desired end product. For example, a curved mold could be used to facilitate machining a curved cranial plate or the like from the preform.

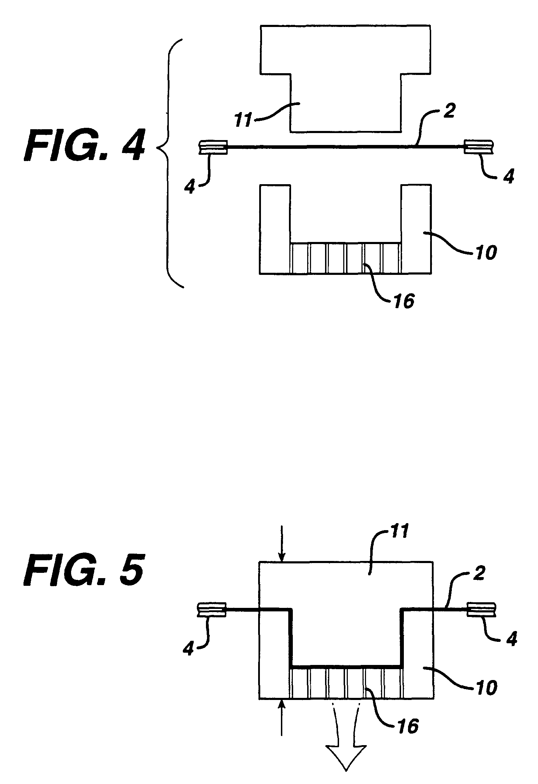

In an alternate method, after the heated sheet is removed from the oven, in a rubbery state, the clamped rubbery sheet is then positioned between a male and a female mold halves. As the mold halves close, the heated or unheated mechanically driven male mold half acts as a plug by pre-stretching the rubbery sheet and preferably a vacuum is applied to the female half of the mold to draw the sheet down into it. The final device thickness depends on the mating tolerances of the two mold halves. The temperature of the male and female halves of the mold is controlled separately. The heated rubbery sheet is oriented as it is stretched and cools during stretching and by its contact with the lower temperatures of the mold halves. Figures 4 and 5 illustrate one simplified embodiment of this process.

In yet another embodiment of the present invention a male mold can be displaced into a heated rubbery sheet to stretch the sheet and provide the final shape for the sheet. This process can also utilize a vacuum drawn through the male mold to facilitate the stretching process. The cooling process of the thermoformed sheet is obtained by contact with colder mold and the thermoformed sheet can be further cooled by forced convection by blowing air.

The device of the present invention can be made from a variety of biocompatible materials and preferably these biocompatible materials are absorbable. Suitable biocompatible absorbable materials from which the plate may be formed include biocompatible absorbable polymers selected from the group consisting of: aliphatic polyesters; polyorthoesters; polyanhydrides; copolymers; blends and combinations thereof.

Examples of suitable organic biocompatible bioabsorbable polymers that could be used include polymers selected from the group consisting of aliphatic polyesters, copoly(ether-esters), polyorthoesters, polyoxaesters, polyamidoesters, polyoxaesters containing amido groups, poly(anhydrides), and blends thereof. For the purpose of this invention aliphatic polyesters include but are not limited to homopolymers and copolymers of lactide (which includes D- and L- lactic acids; D-, L-, and meso lactide), glycolide (including glycolic acid), ε-caprolactone, p-dioxanone (1,4-dioxan-2-one), trimethylene carbonate (1,3-dioxan-2-one), alkyl derivatives of trimethylene carbonate, δ-valerolactone, β-butyrolactone, γ-butyrolactone, ε-decalactone, hydroxybutyrate, hydroxyvalerate, α,α-diethylpropiolactone, ethylene carbonate, ethylene oxalate, 3-methyl-1,4-dioxan-2,5-dione, 3,3-diethyle-1,4-dioxan-2,5-dione, 6,8-dioxabicycloctane-7-one, 2,5-diketomorpholine, 1,4-dioxepan-2-one (including its dimer 1,5,8,12-tetraoxacyclotetradecane-7,14-dione), 1,5-dioxepan-2-one, 6,6-dimethyl-1,4-dioxan-2-one and polymer blends thereof. Preferred are semi-crystalline aliphatic polymers and copolymers (with two or more monomers) to minimize creep in the final product. The starting material would preferably be amorphous but which is crystallizable by orientation and/or heating. Copoly(ether-esters) for the purpose of this invention include those copolyester-ethers described in "Journal of Biomaterials Research", Vol. 22, pages 993-1009, 1988 by Cohn and Younes and Cohn, Polymer Preprints (ACS Division of Polymer Chemistry) Vol. 30(1), page 498, 1989 (e.g. PEO/PLA). Polyanhydrides from diacids of the form HOOC-C₆H₄-O-(CH₂)ₘ-O-C₆H₄-COOH where m is an integer in the range of 2 to 8 and copolymers thereof with aliphatic alpha-omega diacids of up to 12 carbons. Polyoxaesters polyoxaamides and polyoxaesters containing amines and/or amido groups are described in one or more of the following U.S. Patent Nos. 5,464,929; 5,595,751; 5,597,579; 5,607,687; 5,618,552; 5,620,698; 5,645,850; 5,648,088; 5,698,213; and 5,700,583; (which are incorporated herein by reference). Polyorthoesters such as those described by Heller in Handbook of Biodegradable Polymers, edited by Domb, Kost and Wisemen, Hardwood Academic Press, 1997, pages 99-118 (hereby incorporated herein by reference).

The polymers from which the devices are made can have a range of molecular weights as provided by inherent viscosity between about 1 to about 7 deciliters per gram (dL/g), more typically about 1 to about 5 dL/g, and most preferably from about 1.6 to about 3.5 dUg as measured in a 0.1 g/dL solution of hexafluoroisopropanol (HFIP) or chloroform at 25°C. The sheets are processed by compression molding, extrusion or injection molding equipped with dry nitrogen atmospheric chamber(s) at temperatures ranging from about 5°C to about 50°C above their melting temperatures, more preferably 10°C to about 30°C above their melting temperatures.

The device of the present invention can also be made from a blend of bioabsorbable polymers. The components of the blend need to be preferably somewhat immiscible and partially compatible. In the preformed sheet that will be consequently thermoformed, the minor non-continuous phase will exist as spherical or elongated droplets in a matrix (the continuous phase). During the stretching of the heated rubbery sheet made from polymer blends, the droplets of the minor component will elongate to form a drawn structure that may be fibrillar or ribbon like. The fibrils or ribbons formed in situ will be oriented in one or more directions. These fibrils or ribbons can enhance mechanical properties if they are stiffer than the matrix material and can enhance toughness if they are softer than the matrix material.

The orthopedic devices of the present invention can also be made of composites of absorbable materials with reinforcing absorbable glasses or ceramics comprising calcium phosphates and other biocompatible metal oxides (i.e., CaO) and present on form of fibers or particles. The plate of the present invention can further comprise combinations of absorbable ceramics, glasses and polymers.

The device of the present invention can also be made from multi-layered or coextruded materials. The layers can be made from biocompatible materials that may be absorbable and can comprise of polymers; glass or ceramic fiber or particle filled polymers.

In one embodiment of the present invention, the device of the present invention has at least a thickness of 0.3 mm (0.012 inches). Preferably the device has a thickness above 0.5 mm (0.020 inches) and most preferably a thickness above 0.75 mm (0.030 inches).

The devices can have a range of mechanical properties such as, tensile modulus and tensile strength, in the primary draw direction and separate a range of mechanical properties such as, tensile modulus and tensile strength, in a direction transverse to the primary draw direction in the thermoformed part. This has been referred to as just biaxial. In another circumstance the tensile modulus and tensile strength in two draw directions, perpendicular to each other, can be equal. This will be referred to as equi-biaxial.

In one embodiment with polylactide, the tensile modulus of the device can be at least 4.3 GPa and the tensile strength of the device can be at least 80 Mpa, respectively, in at least two orthogonal axes. Preferably, the tensile modulus of the device can be at least 5.0 GPa in one axis and 4.3 GPa in another orthogonal axis and the tensile strength of the device can be at least 100 MPa in one axis and at least 90 MPa in another orthogonal axis. More preferably, the tensile modulus of the device can be at least 6.0 GPa and 4.3 GPa in another orthogonal axis and the tensile strength of the device can be at least 130 MPa in one axis and 85 MPa in another orthogonal axis.

The device of the present invention may be made by using a variety of processing parameters during thermoforming that provide the device with desired properties (such as modulus, tensile strength, elongation etc.) and desired thickness. For example, polylactide pre-formed sheets may be heated to a rubbery state and stretched at a temperature (of the sheet) in the range from about 90 to about 180°C and preferably at a temperature from about 110 to about 160°C and more preferred in the range of from 120 to 140°C. Too low a temperature of the sheet may not provide the sheet with rubbery properties necessary to be stretched to a desired draw ratio. On the other hand, if the temperature is too high, not only will the sheet sag under its own weight but also it will tend to relax and loose the orientation that is responsible for retention of enhanced properties. For example polylactide, the plug temperature should be in the range of from about 30 to about 140 °C, preferably in the range of from about 45 to about 110°C and more preferably in the range of from about 50 to 95 °C. Improper choice of plug temperatures can lead to freezing of the sheet where the plug contacts the sheet and leave chill marks or striations and undesired part thickness for the thermoformed device. For example for polylactide, the mold temperature is typically in the range of from about 25 to about 90 °C, with more typically in the range of from about 35 to about 70°C and most typically in the range of from 40 to 60 °C. Improper mold temperature may cause too fast a freezing of the stretched hot sheet and lock in undesired stresses. It can also cause the orientation to relax or prevent the crystallization of the device made from semi-crystalline polymers.

In general the plug temperature for all aliphatic polyesters is between about 35 °C below its glass transition temperature to about 80 °C above its glass transition temperature with preferred ranges being at the glass transition temperature to 30°C above the glass transition temperature.

In general, the mold temperature of all aliphatic amorphous polyesters is in the range of from about 40 °C below their glass transition temperature to about 40 °C above their glass transition temperature, and preferably the mold temperature will be in range of from 20 °C below their glass transition temperature to 10 °C above their glass transition temperature. In general, the mold temperature of all aliphatic semi-crystalline polyesters is between their glass transition temperature to 100 °C above their glass transition temperature with the preferred range being from 20 to 70 °C above their glass transition temperature.

The draw ratio, as measured by the ratio of the area of the formed device to the original area, is in the range of about 2 to about 10 times, preferably in the range between 3 to 6 and most preferably in the range between 2 to 4. Draw ratios can also be expressed as ratios of initial to final thickness.

Numerous other devices can be machined from the novel device with multi-axial properties that have been described above. Examples of the device include but are not limited to, orthopedics devices (such as join replacement prosthesis, vertebral discs, pins, rods, nails, anchors, cages, screws, and plates for hard and soft tissue fixation); tissue engineering structures (such as tissue scaffolds for in vivo and in vitro tissue regrowth, augmentation and repair); and surgical devices (such as staples, arrows, pledgets, clamps, hooks, buttons, snaps, valves, and clips). In some applications it may be desirable to form a device from sections of thermoformed material with non-equal orientation to impart superior properties along one axis. By selecting the thermoformed materials from specific areas from the thermoformed articles it is possible to make parts with different draw ratios along different axes.

In another embodiment of the present invention, the device with multi-axial properties can be used as a drug delivery matrix. To form the drug delivery matrix one or more of the absorbable polymer that make the preform sheets would be mixed with a therapeutic agent. The variety of different therapeutic agents that can be used in conjunction with the polymers of the present invention is vast. In general, therapeutic agents which may be administered via the pharmaceutical compositions of the invention include, without limitation: antiinfectives such as antibiotics and antiviral agents; analgesics and analgesic combinations; anti-inflammatory agents; hormones such as steroids; bone regenerating growth factors; and naturally derived or genetically engineered proteins, polysaccharides, glycoproteins, or lipoproteins.

The formulations may be made by mixing one or more therapeutic agents with the polymer. The therapeutic agent, may be present as a liquid, a finely divided solid, or any other appropriate physical form. Typically, but optionally, the matrix will include one or more additives, such as diluents, carriers, excipients, stabilizers or the like.

The amount of therapeutic agent will depend on the particular drug being employed and medical condition being treated. Typically, the amount of drug represents about 0.001 percent to about 70 percent, more typically about 0.001 percent to about 50 percent, most typically about 0.001 percent to about 20 percent by weight of the matrix. The quantity and type of polymer incorporated into the drug delivery matrix will vary depending on the release profile desired and the amount of drug employed.

Upon contact with body fluids, the polymer undergoes gradual degradation (mainly through hydrolysis) with concomitant release of the dispersed drug for a sustained or extended period. This can result in prolonged delivery (over, say I to 5,000 hours, preferably 2 to 800 hours) of effective amounts (say, 0.0001 mg/kg/hour to 10 mg/kg/hour) of the drug. This dosage form can be administered as is necessary depending on the subject being treated, the severity of the affliction, the judgment of the prescribing physician, and the like. Following this or similar procedures, those skilled in the art will be able to prepare a variety of formulations.

Additionally, radio-opaque markers may be added to the composite to allow imaging of the composite after implantation.

The following examples are illustrative of the principles and practice of this invention, although not limited thereto. Numerous additional embodiments within the scope and spirit of the invention will become apparent to those skilled in the art.

### Example 1

This example describes the compression molding of a bioabsorbable pre-form, thermoforming of the pre-form and subsequently machining into a device.

50 grams of a poly(L)lactic acid polymer pellets were placed on a metal platen in between square rubber frame 1.5 mm (0.060 inches) thick and measuring 150 mm by 150 mm (6 inches by 6 inches). The inherent viscosity of the polymer was 1.8 dUg as determined in chloroform at 25°C at a concentration of 0.1 g/dL. A second metal platen was placed on top of the first metal platen, the rubber frame and the polymer. The entire assembly was then placed in a compression molding press equipped with a nitrogen chamber to maintain an inert atmosphere and the press had been pre-heated to a temperature of 210°C. After 10 minutes at 210°C, the polymer became molten and a force of 2721 kilogram (6000 pounds) was slowly applied on the platens. The pressure and temperature were maintained for ten minutes. The two platens together with the pressed sheet were removed from the press and transferred to a second press maintained at ambient temperature by circulating cooling water. The plaque, cooled to room temperature and removed from the platen assembly and stored under vacuum.

The 150 mm by 150 mm (6 inches by 6 inches), 1.5 mm (0.060 inches) thick plaque from poly(L)lactic acid was thermoformed by following the process described next. First, the 0.060 inch thick plaque was constrained between two 108 mm by 108 mm (4.25 inch by 4.25 inch) clamps. The entire assembly, consisting of clamp and the plaque was moved in an oven that is heated by a top and a bottom bank of heaters. Both heaters were maintained at 355°C. The plaque was heated for 0.8 minutes. The heated sheet was removed from the oven and was in a rubbery state at a temperature of 130°C. The rubbery sheet was then sealed and restrained against a rim of a female mold, which had a central cavity of 100 mm X 100 mm (3.95 X 3.95 inches) and a depth of 75 mm (3.0 inches). The rubbery sheet was pre-stretched with a heated mechanically driven plug, maintained at 60°C, and into the female mold cavity, maintained at 40°C. Vacuum was then applied to the female mold to pull or draw the stretched sheet against the mold surface until it stopped drawing. The vacuum was applied to the mold for 8 seconds and the stretching was complete and the device in form of a stretched plaque could not deform easily any more as it had cooled considerably. The rapid cooling process was continued by blowing air at ambient temperature. The thermoformed part was then removed from the mold. Samples for tensile testing were machined from the sides of hollow box shaped thermoformed polymer.

Tensile tests were done using an Instron tensile testing machine using a video extensiometer and holding the samples between a fixed and a moving grip, with the speed of the moving grip being 12.5 mm per minute (0.5 inches per minute). The ratio of initial thickness of the plaque to the final thickness of the machined samples is described as the draw ratio or DR.

Table 1 compares the tensile modulus and tensile strength of samples in directions parallel and perpendicular to the primary draw direction which is the along the depth of the female mold (i.e. along the direction of the plug movement). The control sample that was compression molded and has same properties in two perpendicular directions. The data reported is an average of at least 4 samples.

**Table 1 -**

| Properties of thermoformed polylactic acid plaques with IV of 1.8 and initial thickness of 1.5 mm (0.060 inches). | | | | | |
|---|---|---|---|---|---|
| Oven temp. & Heating time | Sample Orientation | Part Thickness mm (inch) | Draw Ratio (DR) | Modulus (Gpa) | Strength (Mpa) |
| Control | None | 1.5 (0.060) | 1 | 3.5 | 70 |
| 355 °C for 0.8 mins | Parallel To Draw | 0.4 (0.016) | 3.75 | 8.0 | 130 |
| | Perpendicular To Draw | 0.4 (0.016) | 3.75 | 7.8 | 122 |

The device machined from the thermoformed preform in the above table demonstrates significant biaxial increase in tensile properties over those of the control.

### Example 2

This example describes the compression molding of a bioabsorbable pre-form of higher initial thickness, thermoforming of the pre-form and subsequently machining into a device.

100 grams of a poly(L)lactic acid polymer pellets were placed on a metal platen in between square rubber frame 3 mm (0.120 inches) thick and measuring 150 mm by 150 mm (6 inches by 6 inches). The inherent viscosity of the polymer was 1.8 dL/g as determined in chloroform at 25°C at a concentration of 0.1 g/dL. A second metal platen was placed on top of the first metal platen, the rubber frame and the polymer. The entire assembly was then placed in a compression molding press equipped with a nitrogen chamber to maintain an inert atmosphere and the press had been pre-heated to a temperature of 210°C. After 10 minutes at 210°C, the polymer became molten and a force of 4082 kilogram (9000 pounds) was slowly applied on the platens. The pressure and temperature were maintained for ten minutes. The two platens together with the pressed sheet were removed from the press and transferred to a second press maintained at ambient temperature by circulating cooling water. The plaque, cooled to room temperature and removed from the platen assembly and stored under vacuum.

The 150 mm by 150 mm (6 inches by 6 inches), 3.0 mm (0.120 inches) thick plaque from poly(L)lactic acid was thermoformed by following the process described next. First, the 3.0 mm (0.120 inches) thick plaque was constrained between two 108 mm by 108 mm (4.25 inch by 4.25 inches) clamps. The entire assembly, consisting of clamp and the plaque was moved in an oven that is heated by a top and a bottom bank of heaters. Both heaters were maintained at 225 °C. The plaque was heated for 2.5 minutes. The heated sheet was removed from the oven and was in a rubbery state at a temperature of 120 °C. The rubbery sheet was then sealed and restrained against a rim of a female mold, which had a central cavity of 100 mm X 100 mm (3.95 X 3.95 inches) and a depth of 75 mm (3.0 inches). The rubbery sheet was pre-stretched with a heated mechanically driven plug, maintained at 60°C, and into the female mold cavity, maintained at 40°C. Vacuum was then applied to the female mold to pull or draw the stretched sheet against the mold surface until it stopped drawing. The vacuum was applied to the mold for 8 seconds and the stretching was complete and the device in form of a stretched plaque could not deform easily any more as it had cooled considerably. The rapid cooling process was continued by blowing air at ambient temperature. The thermoformed part was then removed from the mold. Samples for tensile testing were machined from the sides of hollow box shaped thermoformed polymer.

Tensile tests were done using an Instron tensile testing machine using a video extensiometer and holding the samples between a fixed and a moving grip, with the speed of the moving grip being 12.5 mm per minute (0.5 inches per minute). The ratio of initial thickness of the plaque to the final thickness of the machined samples is described as the draw ratio or DR.

Table 2 compares the tensile modulus and tensile strength of samples in directions parallel and perpendicular to the primary draw direction which is the along the depth of the female mold (i.e. along the direction of the plug movement). The control sample that was compression molded and has same properties in two perpendicular directions. The data reported is an average of at least 4 samples.

**Table 2 -**

| Properties of thermoformed polylactic acid plaques with IV of 1.8 and initial thickness of 3.0 mm (0.120 inches). | | | | | |
|---|---|---|---|---|---|
| Oven Temp. and Heating time | Sample Orientation | Part Thickness Mm (inch) | Draw Ratio (DR) | Modulus (Gpa) | Strength (Mpa) |
| Control | None | 3.0 (0.120) | 1 | 3.5 | 70 |
| 225 °C for 2.5 mins | Parallel To Draw | 0.56 (0.022) | 5.5 | 8.0 | 115 |
| | Perpendicular To Draw | 0.53 (0.021) | 5.7 | 4.5 | 98 |

The device machined from the thermoformed preform in the above table demonstrates significant biaxial increase in tensile properties over those of the control.

### Example 3

This example describes yet another compression molding of a bioabsorbable pre-form, thermoforming of the pre-form and subsequently machining into a device with higher biaxial properties that are equal in both directions or equi-biaxial.

Plaques of thickness 1.5 mm (0.060 inches) and 3.0 mm (0.120 inches) were compression molded using the process described in Example 1 and 2, respectively. The plaques were thermoformed by similar procedures referred to in Examples 1 and 2 with two different forming conditions mentioned in Table 3. The mold temperature was 40°C for both thick and thin sheets while the plug temperature was 50°C for the 3.0 mm (0.120 inch) plaque and 60°C for the 1.5 mm (0.060 inch) plaque. In this case, the samples for tensile testing were machined from the bottom of hollow box shaped thermoformed polymer. The bottom of the hollow formed box showed equal stretch as observed from comparing measured distances marked on the undeformed plaque and the deformed final part.

Tensile tests were done using an Instron tensile testing machine using a video extensiometer and holding the samples between a fixed and a moving grip, with the speed of the moving grip being 12.5 mm per minute (0.5 inches per minute). The ratio of initial thickness of the plaque to the final thickness of the machined samples is described as the draw ratio or DR.

Table 3 compares the tensile modulus and strength of samples in two directions perpendicular to each other and has the same properties in the two perpendicular directions. The control sample was compression molded and has same properties in two perpendicular directions. The data reported below is the average of at least 4 samples.

**Table 3 -**

| Properties of thermoformed polylactic acid plaques with IV of 1.8 having different initial thickness. | | | | | | |
|---|---|---|---|---|---|---|
| Initial Thickness mm (inch) | Oven Temp. and Heating time | Sample Orientation | Part Thickness mm (inch) | Draw Ratio (DR) | Modulus (Gpa) | Strength (Mpa) |
| | Control | None | 1.5 (0.06) | 1 | 3.5 | 70 |
| 1.5 (0.060) | 225°C 2.5 min. | Equi-biaxial Draw | 1.14 (0.045) | 1.3 | 4.5 | 90 |
| 3.0 (0.120) | 225°C 3.5 min. | Equi-biaxial Draw | 0.51 (0.020) | 5.9 | 4.3 | 88 |

The device machined from the thermoformed preform in the above table demonstrates significant biaxial increase in tensile properties over those of the control.

### Example 4

This example describes yet another compression molding of a bioabsorbable pre-form with higher IV, thermoforming of the pre-form and subsequently machining into a device with higher biaxial properties that are equal in both directions or equi-biaxial.

100 grams of a polylactic acid polymer pellets were placed on a metal platen in between square rubber fame of walls of thickness 3 mm (0.120 inches) and measuring 150 mm by 150 mm (6 inches by 6 inches). The inherent viscosity of the polymer was 4.5 dLg as determined in chloroform at 25°C at a concentration of 0.1 g/dL. A second metal platen was placed on top of the first metal platen, the rubber frame and the polymer. The entire assembly was then placed in a compression molding press equipped with a nitrogen chamber to maintain an inert atmosphere and the press had been pre-heated to a temperature of 215°C. After 10 minutes at 215°C, the polymer became molten and a force of 4082 kilogram (9000 pounds) was slowly applied on the platens. The pressure and temperature were maintained for ten minutes. The two platens together with the pressed sheet were removed from the press and transferred to a second press maintained at ambient temperature by circulating cooling water. The plaque, cooled to room temperature and removed from the platen assembly and stored under vacuum.

The 150 mm by 150 mm (6 inches by 6 inches), 3 mm (0.120 inch) thick plaque from polylactic acid was thermoformed by following the process described next. First, the 3 mm (0.120 inch) thick plaque was constrained between two 108 mm by 108 mm (4.25 inch by 4.25 inches) clamps. The entire assembly, consisting of clamp and the plaque was moved in an oven that is heated by a top and a bottom bank of heaters. Both heaters were maintained at 380°C. The plaque was heated for 1.2 minutes. The heated sheet was removed from the oven and was in a rubbery state at a temperature of 140°C. The rubbery sheet was then sealed and restrained against a rim of a female mold, which had a central cavity of 100 mm X 100 mm (3.95 X 3.95 inches) and a depth of 75 mm (3.0 inches). The rubbery sheet was pre-stretched with a heated mechanically driven plug, maintained at 60°C, and into the female mold cavity, maintained at 40°C. Vacuum was then applied to the female mold to pull or draw the stretched sheet against the mold surface until it stopped drawing. The vacuum was applied to the mold for till 8 seconds and the stretching was complete and the device in form of a stretched plaque could not deform easily any more as it had cooled considerably. The rapid cooling process was continued by blowing air at ambient temperature. Samples for tensile testing were machined from the bottom of hollow box shaped thermoformed polymer. The bottom of the hollow formed box showed equal stretch as observed from comparing measured distances marked on the undeformed plaque and the deformed final part.

Tensile tests were done using an Instron tensile testing machine using a video extensiometer and holding the samples between a fixed and a moving grip, with the speed of the moving grip being 12.5 mm per minute (0.5 inches per minute). The ratio of initial thickness of the plaque to the final thickness of the machined samples is described as the draw ratio or DR.

Table 4 compares the tensile modulus and strength of samples in two directions perpendicular to each other and has the same properties in the two perpendicular directions. The control sample was compression molded and has same properties in two perpendicular directions. The data reported below is the average of at least 4 samples.

**Table 4 -**

| Properties of thermoformed polylactic acid plaques with IV of 4.5 and initial thickness of 3 mm (0.120 inches). | | | | | | |
|---|---|---|---|---|---|---|
| Initial Thickness mm (inch) | Oven Temp. and heating time | Sample Orientation | Part Thicknes s mm (inch) | Draw Ratio (DR) | Modulus (Gpa) | Strengt h (Mpa) |
| 3 mm (0.120) | Control | None | 3 mm (0.120) | 1 | 3.7 | 70 |
| 3 mm (0.120) | 380°C for 1.2 min. | Equi-Biaxial Draw | 0.81 mm (0.032) | 3.8 | 4.4 | 80 |

The device machined from the thermoformed preform in the above table demonstrates significant biaxial increase in tensile properties over those of the control.

### Example 5

This example describes yet another compression molding of a bioabsorbable pre-form of higher initial thickness, thermoforming of the pre-form and subsequently machining into a device with higher uniaxial properties.

Plaques of thickness 3 mm (0.120 inch) were compression molded using the process described in Example 2. The plaques were thermoformed by similar procedures referred to in Example 2 with forming conditions of 350°C heating temperature and 1.0 minute heating time. The mold temperature was 40°C for both thick and thin sheet while the plug temperature was 50°C. In this case, the samples for tensile testing were machined from the side of hollow box shaped thermoformed polymer.

Tensile tests were done using an Instron tensile testing machine using a video extensiometer and holding the samples between a fixed and a moving grip, with the speed of the moving grip being 12.5 mm per minute (0.5 inches per minute). The ratio of initial thickness of the plaque to the final thickness of the machined samples is described as the draw ratio or DR.

Table 5 compares the tensile modulus and strength of samples in directions parallel to the primary draw direction which is the along the depth of the female mold or along the direction of the plug movement. The control sample was compression molded and has same properties in two perpendicular directions. The data reported below is the average of at least four samples.

**Table 5 -**

| Properties of thermoformed polylactic acid plaques with IV of 1.8 and initial thickness of 3 mm (0.120 inches). | | | | | |
|---|---|---|---|---|---|
| Oven Temp. and hearing time | Sample Orientation | Part Thickness mm (inch) | Draw Ratio (DR) | Modulus (Gpa) | Strength (Mpa) |
| Control | None | 3.0 (0.120) | 1 | 3.5 | 70 |
| 350°C for 1.0 mins | Parallel To Draw | 0.61 (0.024) | 5 | 6.6 | 137 |

The device machined from the thermoformed preform in the above table demonstrates significant increase in tensile properties in the machined direction over those of the control.

## Claims

1. A thermoformed bioabsorbable polymer preform having orientation in at least one axis suitable for use in medical devices.

2. The thermoformed bioabsorbable polymer preform of claim 1 wherein the preform has a minimum thickness greater than 0.3mm.

3. The thermoformed bioabsorbable polymer preform of claim 1 wherein the preform is oriented in at least two axes.

4. The thermoformed bioabsorbable polymer preform of claim 1 wherein the preformed is made from a bioabsorbable polymer selected from the group consisting of homopolymers and copolymers of lactide, glycolide, glycolic acid, ε-caprolactone, p-dioxanone (1,4-dioxan-2-one), trimethylene carbonate (1,3-dioxan-2-one), alkyl derivatives of trimethylene carbonate, δ-valerolactone, β-butyrolactone, γ-butyrolactone, ε-decalactone, hydroxybutyrate, hydroxyvalerate, α,α-diethylpropiolactone, ethylene carbonate, ethylene oxalate, 3-methyl-1,4-dioxan-2,5-dione, 3,3-diethyle-1,4-dioxan-2,5-dione, 6,8-dioxabicycloctane-7-one, 2,5-diketomorpholine, 1,4-dioxepan-2-one, dimer 1,5,8,12-tetraoxacyclotetradecane-7,14-dione, 1,5-dioxepan-2-one, 6,6-dimethyl-1,4-dioxan-2-one and polymer blends thereof.

5. The thermoformed bioabsorbable polymer preform of claim 1 wherein the bioabsorbable polymer in the preform is semicrystalline.

6. The thermoformed bioabsorbable polymer preform of claim 1 wherein the bioabsorbable polymer in the preform is amorphous.

7. The thermoformed bioabsorbable polymer preform of claim 4 wherein the bioabsorbable polymer contained in the preform has an inherent viscosity of from about 1 to about 7 dL/g as measured in a 0.1 g/dL solution of hexafluoroisopropanol at 25°C.

8. A medical device formed from a thermoformed bioabsorbable polymer preform orientation in at least one axis.

9. The medical device of claim 8 wherein the medical device is selected from the group consisting of joint replacement prosthesis, vertebral discs, pins, rods, nails, anchors, cages, screws, and plates, tissue scaffolds, staples, arrows, pledgets, clamps, hooks, buttons, snaps, valves, and clips.

10. The medical device of claim 8 wherein the thermoformed bioabsorbable polymer contained in the preform also contains a biocompatible, absorbable reinforcing material.

11. A method for manufacturing thermoformed bioabsorbable polymer preforms comprising
(a) heating of a bioabsorbable polymer sheet suitable for use in a medical device until the bioabsorbable polymer sheet is in a rubbery state then
(b) stretching the heated bioabsorbable polymer sheet against a three dimensional mold to provide molecular orientation in at least one axis
(c) cooling the stretched bioabsorbable polymer sheet to retain at least part of the molecular orientation thereby forming a bioabsorbable polymer preform.
